# EUROPEAN PATENT SPECIFICATION

(11) **EP 2 476 719 B1**
(45) Date of publication and mention of the grant of the patent: **02.01.2019**
(21) Application number: 10815167.1
(22) Date of filing: 04.02.2010
(51) Int. Cl.: A61K 8/02, A61K 8/89, A61Q 19/00, C08J 3/12, C08G 77/04, C08G 77/06, C08L 101/00, A61Q 1/02

(54) **IRREGULAR-SHAPED MICROPARTICLES, PROCESS FOR PRODUCTION OF IRREGULAR-SHAPED MICROPARTICLES, AND COSMETICS AND RESIN COMPOSITIONS CONTAINING IRREGULAR-SHAPED MICROPARTICLES**
IRREGULÄR GEFORMTE MIKROPARTIKEL, VERFAHREN ZUR HERSTELLUNG IRREGULÄR GEFORMTER MIKROPARTIKEL SOWIE KOSMETIKARTIKEL UND HARZZUSAMMENSETZUNG MIT DEN IRREGULÄR GEFORMTEN MIKROPARTIKELN
MICROPARTICULES DE FORME IRRÉGULIÈRE, PROCÉDÉ DE PRODUCTION DE MICROPARTICULES DE FORME IRRÉGULIÈRE ET PRODUITS COSMÉTIQUES ET COMPOSITIONS DE RÉSINE CONTENANT DES MICROPARTICULES DE FORME IRRÉGULIÈRE

(30) Priority: 08.09.2009 JP 2009206960
(43) Date of publication of application: 18.07.2012
(73) Proprietor: Takemoto Yushi Kabushiki Kaisha, Gamagori-shi, Aichi 443-8611 (JP)
(72) Inventor: ARATANI Satoshi, Gamagori-shi Aichi 443-8611 (JP); ISHIKAWA Fumiyoshi, Gamagori-shi Aichi 443-8611 (JP); SAITO Chiaki, Gamagori-shi Aichi 443-8611 (JP); YASUI Mamoru, Gamagori-shi Aichi 443-8611 (JP)
(74) Representative: Wallace, Sheila Jane
(86) International application number: PCT/JP2010/051578
(87) International publication number: WO 2011/030569

(56) References cited:
- WO-A1-2006/075711
- JP-A- 61 225 254
- JP-A- 2000 191 788
- JP-A- 2002 179 708
- JP-A- 2007 217 616

## Description

### Background of the Invention

This invention relates to non-spherical fine particles, as well as methods of their production and their use. Fine particles of various substances have been in use in many applications. Their shapes are mostly indefinite, and they are useful even as they are and have been playing their suitable roles as industrial materials. In recent years, however, as the characteristics required of them in various applications become highly advanced, there are beginning to appear many situations where fine particles with controlled shapes are desired. As examples, improvements in usability of cosmetic products, improvements in the optical characteristics in the field of display devices and optical diffusers, and miniaturization in size in the field of electronic components may be considered. This invention relates to non-spherical fine particles of polyhedral shapes with six or more surfaces as a whole, each of them being formed as a concave surface, as well as methods of their production and cosmetic materials and resin compositions containing them.

There have been proposed many kinds of fine particles with controlled shapes such as those made of inorganic and organic materials. As for organic fine particles, Japanese Patent Publications Tokkai 00-103 804 and 11-292907, for example, considered polystyrene fine particles, Japanese Patent Publication Tokkai 11-116649, for example, considered polyurethane fine particles, Japanese Patent Publication Tokkai 11-140181, for example, considered polyimide fine particles, and Japanese Patent Publication Tokkai 61-159427, for example, considered organosilicone fine particles. Since almost all of these prior art fine particles are spherical or nearly spherical, there have in recent years been an increasing number of situations wherein problems were encountered by these prior fine particles not being able to respond to the highly advanced requirements which are imposed upon them recently for purposes of use as explained above. As for fine particles with controlled shapes, Japanese Patent Publication Tokkai 07-157672, for example, proposed hollow fine particles having protrusions and indentations, Japanese Patent Publication Tokkai 2000-191788, for example, proposed nearly spherical fine particles having a large number of small indentations on the surface, Japanese Patent Publication Tokkai 2003-171465, for example, proposed fine particles shaped like a rugby ball, and Japanese Patent Publication Tokkai 2003-128788, for example, proposed semispherical fine particles. Although such prior art fine particles have individually distinctive characteristics, they also have problems of not being able to fully respond to the highly advanced requirements of recent years imposed on them for purposes of use.

### Summary of the Invention

It is therefore an object of this invention to provide non-spherical fine particles which will be capable of responding to the highly advanced requirements of recent years imposed on them for purposes of actual use, including further improvement in feeling, soft focus, coverage and durability related to cosmetic products and in optical characteristics such as total light transmittance and optical diffusible property related to resin molded products, as well as methods of their production and their use.

The inventors herein have carried out investigations in order to solve the aforementioned problems and discovered as a result thereof that what are suitable are non-spherical fine particles of a specific size having a polygonal shape as a whole with six or more surfaces which are each concavely formed.

Thus, this invention relates to non-spherical fine particles characterized as being of a specific size and having a polyhedral shape as a whole with six or more surfaces each formed as a concave surface 11, satisfying all of the following three conditions (1), (2) and (3), as well as methods of producing such non-spherical fine particles and cosmetic products and resin compositions containing such non-spherical fine particles. Condition (1) is that the average value of the maximum external diameters L₁ of the individual non-spherical fine particles should be in the range of 0.1 - 20µm; Condition (2) is that the average value of the ratio between the minimum external diameters L₂ and the maximum external diameters L₁ of the individual non-spherical fine particles should be in the range of 0.60 - 0.97; and Condition (3) is that the average number of concave surfaces 11 of which the ratio of the maximum diameter m₁ with respect the maximum external diameter L₁ is in the range of 0.20 - 0.90 is in the range of 6 - 14 per non-spherical fine particle. In the above, the average values are values based on arbitrarily selected 20 images in a scanning electron microscope photograph and the number of concave surfaces 11 per non-spherical fine particle is calculated as twice the number of concave surfaces 11 observed in this scanning electron microscope photograph.

### Brief Description of the Drawings

Fig. 1 is an enlarged front view for approximately showing a non-spherical fine particle embodying this invention.
Fig. 2 is a scanning electron microscopic photograph with magnification 5000 for showing an example of non-spherical fine particle embodying this invention.
Fig. 3 is a scanning electron microscopic photograph with magnification 2000 for showing an example of non-spherical fine particle embodying this invention.

### Detailed Description of the Invention

Non-spherical fine particles according to this invention are explained first. Non-spherical fine particles according to this invention are each a particle of a specific size having a polyhedral shape as a whole with six or more surfaces each formed as a concave surface 11, satisfying all of the following three conditions (1), (2) and (3) described above. Condition (1) is that the average value of the maximum external diameters L₁ of the individual non-spherical fine particles should be in the range of 0.1 - 20µm, and more preferably in the range of 5 - 15µm. Condition (2) is that the average value of the ratio between the minimum external diameters L₂ and the maximum external diameters L₁ of the individual non-spherical fine particles should be in the range of 0.60 - 0.97, and more preferably in the range of 0.70 - 0.90. Condition (3) is that the average number of concave surfaces 11 of which the ratio of the maximum diameter m₁ with respect to the maximum external diameter L₁ is in the range of 0.20 - 0.90 is in the range of 6 - 14, and more preferably in the range of 10 - 12, per non-spherical fine particle.

Although non-spherical fine particles according to this invention have 6 - 14 and more preferably 10 - 12 concave surfaces 11, on the average per non-spherical fine particle, of which the ratio of the maximum diameter m₁ with respect to the maximum external diameter L₁ is in the range of 0.20 - 0.90, as explained above, those having 3 - 7 concave surfaces 11 on the average per non-spherical fine particle, of which the ratio of the maximum diameter m₁ with respect to the maximum external diameter L₁ is in the range of 0.50 - 0.90 are more preferred.

In the above, the average values are values obtained from arbitrarily selected 20 images of particles in a scanning electron microscope photograph, and the number of concave surfaces 11 per non-spherical fine particle is to be twice the number of concave surfaces 11 observed in this scanning electron microscope photograph.

As will be explained in detail below, non-spherical fine particles of this invention have many characteristics that are useful as materials for cosmetic products and resin compositions, one of these being the magnitude of oil absorption. It is preferable that this magnitude be in the range of 70 - 170ml/100g.

The non-spherical fine particles of this invention shaped as explained above comprise siloxane units comprising a polysiloxane cross-link structure. This polysiloxane cross-link structure is a structure having siloxane units forming a three-dimensional network structure. The units comprise siloxane units shown by SiO₂, siloxane units shown by R¹SiO_{1.5}, and siloxane units shown by R²R³SiO, where R¹, R² and R³ are each alkyl group with 1-4 carbon atoms or phenyl group.

Examples of R¹, R² and R³ include alkyl groups with 1-4 carbon atoms and phenyl groups such as methyl group, ethyl group, propyl group and butyl group, but methyl group is preferable. Thus, although examples of siloxane units R¹SiO_{1.5} and R²R³SiO include methyl siloxane unit, ethyl siloxane unit, propyl siloxane unit, butyl siloxane unit and phenyl siloxane unit, methyl siloxane unit is preferable among these examples.

The polysiloxane cross-link structure of the present invention comprises siloxane units SiO₂ in an amount of 30-50 molar %, siloxane units R<1>SiO1.5 in an amount of 40-60 molar % and siloxane units R²R³SiO in an amount of 5-20 molar % such that the total would be 100 molar %.

Next, the method of producing non-spherical fine particles according to this invention will be described. Non-spherical fine particles according to this invention as described above are obtained by using silanol group forming silicide SiX₄ in an amount of 30-50 molar %, silanol group forming silicide R⁴SiY₃ in an amount of 40-60 molar % and silanol group forming silicide R⁵R⁶SiZ₂ in an amount of 5-20 molar % such that the total would be 100 molar %, where R⁴,R⁵ and R⁶ are each alkyl group with 1-4 carbon atoms or phenyl group, and X, Y and Z are each alkoxy group with 1-4 carbon atoms, alkoxyethoxy group having alkoxy group with 1-4 carbon atoms, acyloxy group with 2-4 carbon atoms, N,N-dialkylamino group having alkyl group with 1-4 carbon atoms, hydroxyl group, halogen atom or hydrogen atom, obtained by firstly generating silanol compounds by causing silanol group forming silicide SiX₄ to contact water in the presence of an acidic catalyst for hydrolysis and then causing a condensation reaction of these silanol compounds with silanol group forming silicides R⁴SiY₃ and R⁵R⁶SiZ₂ in an aqueous condition in the presence of an acidic catalyst and a nonionic surfactant.

R⁴, R⁵ and R⁶ are alkyl groups with 1-4 carbon atoms and phenyl groups, among which methyl group is preferable.

Silanol group forming silicide SiX₄ is a compound which eventually forms siloxane unit SiO₂. X in SiX₄ is selected from (1) alkoxy groups with 1-4 carbon atoms such as methoxy group and ethoxy group, (2) alkoxyethoxy groups having alkoxy group with 1-4 carbon atoms such as methoxyethoxy group and butoxyethoxy group, (3) acyloxy groups with 2-4 carbon atoms such as acetoxy group and propyoxy group, (4) N,N-dialkylamino groups having alkyl group with 1-4 carbon atoms such as dimethylamino group and diethylamino group, (5) hydroxyl group, (6) halogen atoms such as chlorine atom and bromine atom, and (7) hydrogen atom.

Specific examples of silanol group forming silicide SiX₄ include tetramethoxy silane, tetraethoxy silane, tetrabutoxy silane, trimethoxyethoxy silane, tributoxyethoxy silane, tetraacetoxy silane, tetrapropyoxy silane, tetra(dimethylamino) silane, tetra(diethylamino) silane, tetrahydroxy silane, chlorosilane triol, dichlorodisilanol, tetrachlorosilane, and chlorotrihydrogen silane, among which tetramethoxy silane, tetraethoxy silane and tetrabutoxy silane are preferred.

Silanol group forming silicide R⁴SiY₃ is a compound which eventually forms siloxane units R¹SiO_{1.5}. Y in R⁴SiY₃ is similar to X in SiX₄ and R⁴ in R⁴SiY₃ is similar to R¹ in R¹SiO_{1.5}.

Examples of silanol group forming silicide R⁴SiY₃ include, as explained above regarding R¹ in siloxane units R¹SiO_{1.5}, those silanol group forming silicides which eventually form methyl siloxane unit, ethyl siloxane unit, propyl siloxane unit, butyl siloxane unit, or phenyl siloxane unit such as methyltrimethoxy silane, ethyltriethoxy silane, propyltributoxy silane, butyltributoxy silane, phenyltris(2-methoxyethoxy)silane, methyltris(2-butoxyethoxy)silane, methyltriacetoxysilane, methyltripropyoxy silane, methylsilanetriol, methylchlorodisilanol, methyltrichlorosilane, and methyltrihydrogen silane, but those silanol group forming silicides which come to form methyl siloxne are preferred.

Silanol group forming silicide R⁵R⁶SiZ₂ is a compound which eventually forms siloxane units R²R³SiO. Z in R⁵R⁶SiZ₂ is similar to X in SiX₄, and R⁵ and R⁶ in R⁵R⁶SiZ₂ are similar to R² and R³ in R²R³SiO.

Examples of silanol group forming silicide R⁵R⁶SiZ₂ include, as explained above regarding R² and R³ in siloxane units R²R³SiO, those silanol group forming silicides which eventually form dimethyl siloxane unit, diethyl siloxane unit, dipropyl siloxane unit, dibutyl siloxane unit or methylphenyl siloxane unit such as dimethyldimethoxy silane, diethyldiethoxy silane, dipropyldibutoxy silane, dibutyldimethoxy silane, methylphenyl methoxyethoxy silane, dimethylbutoxyethoxy silane, dimethyldiacetoxy silane, dimethyldipropyoxy silane, dimethyl silane diol, dimethylchlorosilanol, dimethyldicholosilane, and dimethyldihydrogen silane, but those which eventually form dimethyl siloxane unit are preferred.

For producing non-spherical hollow fine particles embodying this invention, silanol group forming silicide SiX₄ in an amount of 30-50 molar %, silanol group forming silicide R⁴SiY₃ in an amount of 40-60 molar % and silanol group forming silicide R⁵R⁶SiZ₂ in an amount of 5-20 molar % are used such that their total is 100 molar %. Silanol group forming silicide SiX₄ is firstly caused to undergo hydrolysis by contacting water in the presence of an acidic catalyst so as to produce a silanol compound. A known kind of acidic catalyst may be employed for the hydrolysis. Examples of such a known acidic catalyst include inorganic acids such as hydrochloric acid, sulfuric acid, nitric acid and phosphoric acid and organic acids such as formic acid, acetic acid, citric acid, methane sulfonic acid, toluene sulfonic acid, dodecyl benzene sulfonic acid, decyl sulfate, dodecyl sulfate, tetradecyl sulfate and hexadecyl sulfate. It is generally preferable that the acidic catalyst to be made present for the hydrolysis be at a concentration of 0.001-0.5 mass % with respect to the total silanol group forming silicides used in the reaction.

Next, the silanol compound generated as explained above and silanol group forming silicides R⁴SiY₃ and R⁵R⁶SiZ₂ are caused to undergo a condensation reaction in an aqueous condition in the presence of an acidic catalyst and a nonionic surfactant. As the acidic catalyst for the condensation reaction, as in the case of that for the hydrolysis, those of a known kind can be use, and it is preferable to cause it to be present at a concentration of 0.001-0.5 mass % with respect to the total amount of the silanol group forming silicides used for the reaction.

As the nonionic surfactant to be added to the reacting system together with the acidic catalyst, too, those of a known kind may be used. Examples of such nonionic surfactant include those with oxyalkylene groups comprising oxyethylene groups and/or oxypropylene groups such as polyoxyalkylene alkylether, polyoxyalkylene alkylphenylether, polyoxyalkylene alkylesters and castor oil polyoxyalkylene adducts, having polyoxyalkylene groups in the molecule. It is preferable to cause the nonionic surfactant to be made present at a concentration of 0.001-0.55 mass % with respect to the total amount of the silanol group forming silicides used for the reaction.

The mass ratio of water to the total amount of the silanol group forming silicides is normally 10/90 - 70/30. The amount of the catalyst to be used varies according to its kind as well as to the kind of the silanol group forming silicide but it is preferably 1 mass % or less with respect to the total amount of the silanol group forming silicides. The reaction temperature is usually 0 - 40°C but preferably 30°C or less in order to avoid any instantly occurring condensation reaction of the silanol which has been generated by the hydrolysis.

The reaction liquid containing the silanol compounds which has been generated as described above is provided continuously to the condensation reaction to generate non-spherical fine particles of this invention. By the production method according to the present invention, since the acidic catalyst for the hydrolysis can be used also as the acidic catalyst for the condensation reaction, the reaction liquid containing silanol compounds generated by the hydrolysis can be used for the condensation reaction either directly, by further adding an acidic catalyst, or after deactivating or removing the acidic catalyst remaining in the reaction liquid and the silanol group forming silicides which have not reacted. In either situation, the amount of water used is controlled such that the solid concentration of the non-spherical fine particles in the aqueous suspension will be 2-20 mass %, or preferably 5-15 mass %.

Non-spherical fine particles of this invention can be used as an aqueous material with the solid component adjusted to be 30-70 mass % by separating from the aforementioned aqueous suspension, say, by passing through a metallic net and through centrifugation or pressure filtration, or they may be used in a dried form. The dried form can be obtained by passing the aqueous suspension through a metallic net, dehydrating by centrifugation or pressure filtration and drying the dehydrated product by heating at 100-250°C. It can also be obtained by a method of directly heating and drying the aqueous suspension by a spray drier at 100-250°C. Such dried materials are preferably crushed, for example, by using a jet mill.

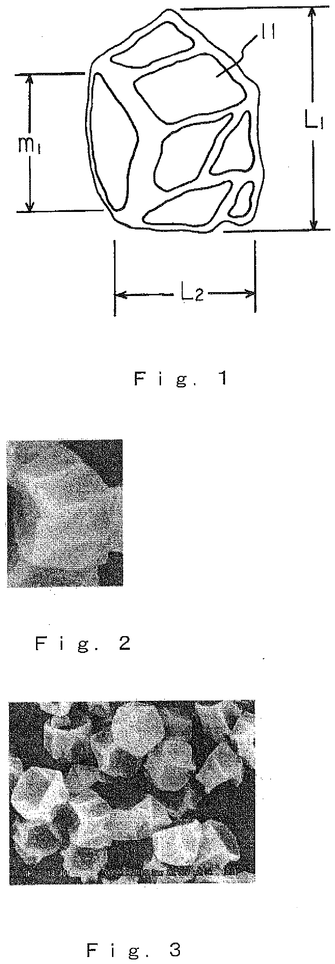

Non-spherical fine particles of this invention thus obtained, as shown in Figs. 1-3, are polyhedral in shape as a whole, having six of more surfaces each of which is formed as a concave surface 11, and satisfying all of the conditions (1), (2) and (3) shown above.

Lastly, cosmetic materials and resin compositions according to this invention are explained. Cosmetic materials according to this invention are characterized as containing those non-spherical fine particles of this invention described above in an amount of 0.1 - 10 mass %.

Cosmetic materials according to this invention are superior in terms of their soft focus effect with reduced roughness and glare, improved coverage of skin freckles and spread on and fitness to the skin due to the superior optical characteristics and high oil absorption of the non-spherical fine particles of this invention when used as a basic cosmetic article in a liquid, cream or press form or as an ingredient of a make-up cosmetic article and hence are useful against falling make-up due to sebum.

Other materials that can be used together with non-spherical fine particles of this invention when a cosmetic material of this invention is produced include body pigments, white pigments, pearl pigments, color pigments (dyes), binding ointments, water, surfactants, thickeners, preservatives, antioxidants, and perfumes. Cosmetic materials of this invention can be prepared by any known method for uniformly dispersing such other materials together with non-spherical fine particles of this invention.

Resin compositions according to this invention are characterized as containing non-spherical fine particles of this invention described above in an amount of 0.1 - 10 mass % and are useful for improving characteristics of various molded resin products obtained therefrom. In the case of molded resin products requiring advanced optical characteristics such as illumination and display devices, for example, products with high optical transmissivity and haze and improved optical diffusibility are becoming desired due to the requirement for highly effective use of light. Resin compositions according to this invention are useful for obtaining molded resin products satisfying such requirement.

The present invention, as described above, can sufficiently respond to the requirements of recent years such as further improvement in feeling, soft focus, coverage and durability regarding cosmetic products and further improvement in optical characteristics such as total light transmittance and optical diffusibility regarding molded resin products.

Next, the invention will be described in terms of test examples but they are not intended to limit the scope of the invention. In the following test examples and comparison examples, "part" will mean "mass part" and "%" will mean "mass %".

A non-spherical fine particle of this invention schematically shown in Fig. 1 is a non-spherical particle having an overall shape of a polyhedron with six or more surfaces each of which is formed as a concave surface 11, satisfying Condition (1) that the average value of the maximum external diameters L₁ of the individual non-spherical fine particles should be in the range of 0.1 - 20µm, Condition (2) that the average value of the ratio between the minimum external diameters L₂ and the maximum external diameters L₁ of the individual non-spherical fine particles should be in the range of 0.60 - 0.97, and Condition (3) that the average number of concave surfaces 11 of which the ratio of the maximum diameter m₁ with respect the maximum external diameter L₁ is in the range of 0.20 - 0.90 is in the range of 6 - 14 per non-spherical fine particle. Such non-spherical fine particles are actually shaped as shown in the scanning electron microscopic photographs of Figs. 2 and 3.

### Part 1: Synthesis of non-spherical fine particles

### Test Example 1 (Synthesis of non-spherical fine particles (P-1))

Ion exchange water 2000g was taken into a reactor vessel and 30% aqueous solution of hydrochloric acid 0,15g was added thereinto and dissolved. Tetraethoxy silane 270.0g (1.30 mols) was further added to carry out hydrolysis with stirring at 15°C for 60 minutes. An aqueous solution was separately prepared in another reactor vessel by dissolving α-(p-nonylphenyl)-ω-hydroxypolyoxy ethylene (10 oxyethylene units, hereinafter n=10) 0.73g and 30% aqueous solution of hydrochloric acid 2.82g in ion exchange water 350g and cooled to 10°C, and the aforementioned hydrolysate solution adjusted to the same temperature was gradually dropped into it with stirring. Methyltrimethoxy silane 277.4g (2.04 mols) and dimethyldimethoxy silane 44.4g (0.37 moles) were further added and the whole was left quietly for one hour while being maintained at 13-15°C. After it was maintained at the same temperature for 4 hours, it was heated to 60°C for a reaction at the same temperature for 5 hours to obtain a white suspension. After the suspension thus obtained was maintained quietly overnight, the white solid phase obtained by removing the liquid phase by decantation was washed with water by a usual method and dried to obtain non-spherical fine particles (P-1). Regarding non-spherical fine particles (P-1), observations and measurements by a scanning electron microscope photograph image as explained below, elemental analysis, inductively coupled plasma spectrometry, FT-IR spectrometry and NMR spectrometry were carried out. As a result, it was ascertained that non-spherical fine particles (P-1) were non-spherical fine particles having an overall shape of a polyhedron with six or more surfaces each of which is formed as a concave surface 11, the average value of the maximum diameters (L₁) of the non-spherical fine particles being 7.8µm and the ratio (L₂/L₁) of the average of the minimum diameters (L₂) to the maximum diameters (L₁) being 0.83. The average number of concave surfaces 11 per non-spherical fine particle with the ratio (m₁/L₁) between the maximum diameter (m₁) of concave surfaces and the maximum external diameter (L₁) in the range of 0.2-0.9 was 11 and the average number of concave surfaces 11 per non-spherical fine particle with the ration m₁/L₁ in the range of 0.5 - 0.9 was 5. The non-spherical fine particles hereby obtained had siloxane units SiO₂ in the amount of 35 molar %, siloxane units R¹SiO_{1.5} in the amount of 55 molar % and siloxane units R²R³SiO in the amount of 10 molar % such that they were together 100 molar %, and their oil absorption was 155ml/100g. Observations and measurements by using scanning electron microscope photograph, measurements of oil absorption and analyses of constituent siloxane units were carried out as follows.

### Observations and measurements by scanning electron microscope photograph

A scanning electron microscope (SEMEDX Type N, produced by Hitachi, Ltd.) was used to observe at magnifications of 2000 - 5000 to obtain an image. Arbitrarily 20 non-spherical fine particles (P-1) were selected out of this image and observed, and their maximum diameters L₁ and their minimum diameters L₂ were measured to obtain average values of L₁ and ratio L₂/L₁. From these selected 20 images, the numbers per particle of concave surfaces with the ratio m₁/L₁ between its maximum diameter m₁ and the maximum external diameter L₁ within the range of 0.20 - 0.90 and their average value, as well as the numbers per particle of concave surfaces with the ratio m₁/L₁ within the range of 0.50 - 0.90 and their average were obtained.

### Measurement of oil absorption

Measurements were made according to JIS-K5101-13-1 (2004).

### Analysis of constituent siloxane units of non-spherical fine particles (P-1)

Non-spherical fine particles (P-1) 5g were accurately measured and added to 0.05N aqueous solution of sodium hydroxide 250ml to extract all of the hydrolyzable groups in the non-spherical hollow fine particles. Non-spherical hollow fine particles were separated by ultra-centrifugation from the extraction-processed liquid, and after the separated non-spherical hollow fine particles were washed with water and dried at 200°C for 5 hours, elemental analysis, inductively coupled plasma spectrometry and FT-IR spectrometry were carried out on them to measure total carbon content and the amount of contained silicon, and silicon-carbon bonding and silicon-oxygen-silicon bonding were examined. Based on these analyzed values, integrated values of NMR spectrum of CP/MAS on solid ²⁹Si, the number of carbon atoms in R⁴ of silanol group forming silicide R⁴SiY₃, and the numbers of carbon atoms in R⁵ and R⁶ of silanol group forming silicide R⁵R⁶SiZ₂, the ratios of siloxane units SiC₂, siloxane units R¹SiO_{1.5} and siloxane units R²R³SiO were calculated.

### Test Examples 2-7 (Syntheses of non-spherical hollow fine particles (P-2) - (P-7))

Non-spherical fine particles (P-2) - (P-7) were synthesized as done in Test Example 1 and observations, measurements and analyses similar to those done in Test Example 1 were carried out.

### Comparison Example 1 (Synthesis of fine particles (R-1)

Ion exchange water 2000g, acetic acid 0.12g and 10% aqueous solution of dodecylbenzene sodium sulfonate 7.1g were taken into a reactor vessel and made into a uniform aqueous solution. Tetraethoxy silane 270.0g (1.30 mols), methyltrimethoxy silane 277.7g (2.04 mols) and dimethyldimethoxy silane 44.4g (0.37 mols) were added to this aqueous solution to carry out hydrolysis at 30°C for 30 minutes. Next, ion exchange water 700g and 30% aqueous solution of sodium hydroxide 1.86g were added into another reactor vessel to prepare a uniform aqueous solution. While this aqueous solution was being stirred, the aforementioned hydrolyzed liquid was gradually added to carry out a reaction at 15°C for 5 hours and further for 5 hours at 80°C to obtain a suspension. After this suspension was left quietly overnight, its liquid phase was removed by decantation, the white solid phase thus obtained was washed with water by a usual method and dried to obtain fine particles (R-1). Observations, measurements and analyses similar to those in Test Example 1 were carried out on fine particles (R-1). Details of non-spherical hollow fine particles, etc. of the examples synthesized as above are shown together in Tables 1-3.

**Table 1**

| | Type of particles | Silanol group forming silicide SiX₄ | Silanol group forming silicide R⁴SiY₃ | Silanol group forming silicide R⁵R⁶SiZ₂ | Catalyst for hydrolysis | Catalyst for condensation reaction | Surfactant |
|---|---|---|---|---|---|---|---|
| TE-1 | P-1 | SM-1 / 35 | SM-3 / 55 | SM-6 / 10 | CA-1 / 0.017 | CA-1 / 0.143 | N-1 / 0,124 |
| TE-2 | P-2 | SM-1 / 40 | SM-3 / 45 | SM-6 / 15 | CA-1 / 0.075 | CA-1 / 0.066 | N-1 / 0.035 |
| TE-3 | P-3 | SM-1 / 45 | SM-3 / 45 | SM-6 / 5 | CA-2 / 0.022 | CA-2 / 0.210 | N-2 / 0.008 |
| | | | SM-4 / 5 | | | | |
| TE-4 | P-4 | SM-2 / 35 | SM-3 / 49 | SM-6 / 10 | CA-2 / 0.035 | CA-2 / 0.115 | N-2 / 0.016 |
| | | | SM-5 / 6 | | | | |
| TE-5 | P-5 | SM-2 / 40 | SM-3 / 45 | SM-6 / 5 | CA-1 / 0.008 | CA-2 / 0.015 | N-2 / 0.310 |
| | | | | SM-7 / 10 | | | |
| TE-6 | P-6 | SM-2 / 45 | SM-3 / 41 | SM-7 / 5 | CA-1 / 0.400 | CA-2 / 0.020 | N-1 / 0.025 |
| | | | SM-4 / 9 | | | | |
| TE-7 | P-7 | SM-2 / 38 | SM-3 / 37 | SM-6 / 8 | CA-2 / 0,030 | CA-1 / 0.011 | N-1 / 0,025 |
| | | | SM-5 / 12 | SM-7 / 5 | | | |
| CE-1 | R-1 | SM-1 / 35 | SM-3 / 55 | SM-6 / 10 | CA-2 / 0.020 | CA-3 / 0.094 | A-1 / 0.120 |

| | | | | | | | |
|---|---|---|---|---|---|---|---|
| In Table 1: TE: Test Example CE: Comparison Example Silicides are shown for "Type / Amount of use" Catalysts and surfactants are shown for "Type / Concentration" Amount of use: Molar % with respect to the total 100% of silanol group forming silicides used as material Concentration of catalyst for hydrolysis: Concentration (mass %) of catalyst with respect to silanol group forming silicide SiX₄ Concentration of catalyst for condensation reaction: Concentration (mass %) of catalyst with respect to the total of silanol group forming silicides used as material Concentration of surfactant: Concentration of surfactant (mass %) with respect to the total of silanol group forming silicides used as material SM-1: Tetraethoxy silane SM-2: Tetramethoxy silane SM-3: Methyltrimethoxy silane SM-4: Propyltributoxy silane SM-5: Phenyltrimethoxy silane SM-6: Dimethyldimethoxy silane SM-7: Methylphenylmethoxyethoxy silane CA-1: Hydrochloric acid CA-2: Acetic acid CA-3: Sodium hydroxide N-1: α-(p-nonylphenyl)-ω-hydroxypolyoxy ethylene (n=10) N-2: α-dodecyl-ω-hydroxypolyoxy ethylene (n=12) A-1: Dodecylbenzene sodium sulfonate | | | | | | | |

**Table 2**

| | Type of particles | Siloxane units SiO₂ | | Siloxane units R¹SiO_{1.5} | | Siloxane units R²R³SiO | | Shape as a whole | Surface condition |
|---|---|---|---|---|---|---|---|---|---|
| | | Type | Ratio | Type | Ratio | Type | Ratio | | |
| TB-1 | P-1 | S-1 | 35 | S-2 | 55 | S-5 | 10 | 1* | 3* |
| TE-2 | P-2 | S-1 | 40 | S-2 | 45 | S-5 | 15 | 1* | 3* |
| TE-3 | P-3 | S-1 | 45 | S-2 | 45 | S-5 | 5 | 1* | 3* |
| | | | | S-3 | 5 | | | | |
| TE-4 | P-4 | S-1 | 35 | S-2 | 49 | S-5 | 10 | 1* | 3* |
| | | | | S-4 | 6 | | | | |
| TE-5 | P-5 | S-1 | 40 | S-2 | 45 | S-5 | 5 | 1* | 3* |
| | | | | | | S-6 | 10 | | |
| TE-6 | P-6 | S-1 | 45 | S-2 | 41 | S-6 | 5 | 1* | 3* |
| | | | | S-3 | 9 | | | | |
| TE-7 | P-7 | S-1 | 38 | S-2 | 37 | S-5 | 8 | 1* | 3* |
| | | | | S-4 | 12 | S-6 | 5 | | |
| CP-1 | R-1 | S-1 | 35 | S-2 | 55 | S-5 | 10 | 2* | 4* |

| | | | | | | | | | |
|---|---|---|---|---|---|---|---|---|---|
| In Table 2: S-1: Anhydrous silicic acid unit S-2: Methyl siloxane unit S-3: Propyl siloxane unit S-4: Phenyl siloxane unit S-5: Dimethyl siloxane unit S-6; Methylphenyl siloxane unit Ratio: Molar % 1*: Spindle shape as a whole with a plurality of concave parts on the surface and a crack in the direction of the major axis 2* Shape of a rugby ball as a whole with a crack on peripheral surface in the longitudinal direction 3* Polyhedral with each surface formed as a concave surface 4* Smooth surfaces | | | | | | | | | |

**Table 3**

| | Type of particles | Shape | | Average number of concave surfaces 11 | | Oil absorption (ml/100g) |
|---|---|---|---|---|---|---|
| | | Average value of L₁ in µm | Average of ratios L₂/L₁ | A | B | |
| TE-1 | P-1 | 7.8 | 0.83 | 11 | 5 | 155 |
| TE-2 | P-2 | 10.5 | 0.73 | 10 | 7 | 163 |
| TE-3 | P-3 | 18.2 | 0.64 | 13 | 4 | 132 |
| TE-4 | P-4 | 0.5 | 0,80 | 10 | 5 | 124 |
| TE-5 | P-5 | 14.9 | 0.95 | 8 | 6 | 102 |
| TE-6 | P-6 | 9.5 | 0.92 | 9 | 3 | 90 |
| TE-7 | P-7 | 0.9 | 0.67 | 7 | 3 | 78 |
| CE-1 | R-1 | 2.5 | - | - | - | 42 |

| | | | | | | |
|---|---|---|---|---|---|---|
| In Table 3: L₁: Maximum external diameter of individual non-spherical fine particles L₂: Minimum external diameter of individual non-spherical fine particles A: Average number of concave surfaces 11 per non-spherical fine particle with m₁/L₁ within the range of 0.20-0.90. B: Average number of concave surfaces 11 per non-spherical fine particle with m₁/L₁ within the range of 0.50-0.90. | | | | | | |

### Part 2

In order to examine the practicality of non-spherical fine particles of this invention as cosmetic products, foundations were prepared and evaluated as follows.

### Preparation of foundations

Foundations comprising a composition as shown in Table 4 were prepared. The preparation was made by using a mixer to mix constituents numbered 1-7 at the mass ratios shown in Table 4, and a mixture which had been separately prepared by taking the components preliminarily numbered 8-12 at the mass ratios shown in Table 4 and had been heated to 40°C was added thereinto and mixed again. After this mixture was left to be cooled, it was crushed and molded to prepare the foundations.

**Table 4**

| Number | Composition | Mass ratio |
|---|---|---|
| 1 | Non-spherical fine particles | 7 |
| 2 | Titanium oxide | 10 |
| 3 | Talc | 20 |
| 4 | Sericite | 35 |
| 5 | Red iron oxide | 0.45 |
| 6 | Yellow iron oxide | 1 |
| 7 | Black iron oxide | 0.05 |
| 8 | Fluidic paraffin | 10 |
| 9 | Octylmethyl cyclotetra siloxane | 5 |
| 10 | Polyoxyalkylene modified silicone | 1.5 |
| 11 | Sorbitan aliphatic ester | 5 |
| 12 | Myristyl alcohol | 5 |

### Evaluation

The foundations described above were evaluated individually by twenty female panelists regarding their usability (extensions and expansions at the time of use) and feeling (stickiness, roughness and durability) according to the evaluation standards shown in Table 5. The results which have been rounded off are shown in Table 6.

**Table 5**

| Point | Evaluation standard |
|---|---|
| 4 | Very good |
| 3 | Good |
| 2 | Somewhat poor |
| 1 | Poor |

**Table 6**

| | Type of non-spherical fine particles | Evaluation | | | |
|---|---|---|---|---|---|
| | | Extensions and expansions | Stickiness | Roughness | Durability |
| TE-8 | P-1 | 4 | 4 | 4 | 4 |
| TE-9 | P-2 | 4 | 4 | 4 | 4 |
| TE-10 | P-3 | 3 | 4 | 4 | 3 |
| TE-11 | P-4 | 4 | 3 | 4 | 3 |
| TE-12 | P-5 | 4 | 3 | 3 | 3 |
| TE-13 | P-6 | 3 | 4 | 3 | 3 |
| TE-14 | P-7 | 3 | 3 | 3 | 3 |
| CE-2 | R-1 | 3 | 2 | 3 | 2 |
| CE-3 | R-2 | 2 | 1 | 1 | 3 |
| CE-4 | R-3 | 1 | 1 | 1 | 2 |
| CE-5 | R-4 | 1 | 1 | 1 | 1 |

| | | | | | |
|---|---|---|---|---|---|
| In Table 6: P-1 - P-7, R-1: Non-spherical fine particles synthesized in Part 1 R-2: Spherical silicone fine particles (Tospearl 120 (tradename) produced by Toshiba Silicone Co., Ltd.) R-3: Spherical vinyl fine particles (Ganz Pearl GSM (tradename) produced by Ganz Chemical Co., Ltd.) R-4: Talc | | | | | |

### Part 3

In order to examine the utility of non-spherical fine particles of this invention as resin composition, sample resin compositions were prepared and evaluated as follows,

### Preparation of resin compositions and production of test plates

Non-spherical hollow fine particles, etc. (0.7 parts) were added to polycarbonate resin (Panlite K1285 (tradename) produced by Teijin Chemicals, Ltd.) (100 parts) and after they were mixed together, they were melted and kneaded together at resin temperature of 280°C by using a biaxial extruder (40mmΦ) equipped with vent to obtain pellets of resin composition by extrusion. Next, these pellets of resin composition were molded by using an injection molding machine at cylinder temperature of 230°C and mold temperature of 60°C and test plates of 200 x 500mm with thickness 3mm were produced

### Evaluation of resin compositions

Total light transmittance and haze were measured as follows by using the test pieces described above. The results are shown in Table 7.

Total light transmittance and haze were measured according to JIS-K7105 (1981) by using NDH-2000 (tradename) produced by Nippon Denshoku Industries Co., Ltd.

For the measurement of heat-resistant colorability, the aforementioned test piece was cut to produce 200 x 200mm sample films. The sample films thus cut out were placed inside a heated air circulating oven at temperature of 80°C and maintained there for 180 minutes, Thereafter, the degree of coloration by heating was measured in terms of the b-value by using a color meter (CR-300 (tradename) produced by Minolta Co., Ltd.). The value of Δb was calculated according to JIS-Z8729 (2004) from the formula Δb = b₂ - b₁ where b₁ is the b-value of the sample film before the heat treatment and b₂ is the b-value of the sample film after the heat treatment,

**Table 7**

| | Type of non-spherical fine particles | Total light transmittance | Haze | Heat-resistant colorability |
|---|---|---|---|---|
| TE-15 | P-1 | 92.6 | 89.3 | 0.0 |
| TE-16 | P-2 | 92.9 | 90.2 | 0.0 |
| TE-17 | P-3 | 88.6 | 86.0 | 0.1 |
| TE-18 | P-4 | 88.4 | 85.4 | 0.2 |
| TE-19 | P-5 | 86.8 | 84.9 | 0.2 |
| TE-20 | P-6 | 86.5 | 84.0 | 0.2 |
| TE-21 | P-7 | 84.2 | 83.1 | 0.3 |
| CE-6 | R-1 | 81.6 | 80.1 | 0.2 |
| CE-7 | R-2 | 82.1 | 79.2 | 0.2 |
| CE-8 | R-3 | 80.8 | 79.4 | 4.0 |
| CE-9 | R-5 | 78.4 | 76.7 | 2.5 |

| | | | | |
|---|---|---|---|---|
| In Table 7: P-1 - P-7, R-1 - R-3: Non-spherical fine particles, etc. described in Table 6 R-5: Calcium carbonate Total light transmittance: % Heat-resistant colorability: Value of Δb | | | | |

The results shown in Table 6 and 7 clearly indicate that non-spherical fine particles of this invention can fully respond to the advanced requirements of recent years both as cosmetic materials and as resin compositions.

## Claims

1. Non-spherical fine particles each having a polyhedral general shape with six or more surfaces each of which is formed as a concave surface,
wherein the maximum external diameters L₁ of the non-spherical fine particles have an average value in the range of 0.1 - 20µm,
wherein the ratios between the minimum external diameters L₂ and the maximum external diameters L₁ of the individual non-spherical fine particles have an average value in the range of 0.60 - 0.97,
wherein said non-spherical fine particles have an average number per particle of 6 - 14 concave surfaces of which the ratio of the maximum diameter m₁ with respect to the maximum external diameter L₁ is in the range of 0.20 - 0.90, and
wherein the average values are values obtained from arbitrarily selected 20 of a scanning electron microscope photograph image of said non-spherical fine particles and the number of concave surfaces per non-spherical fine particle is defined as being twice the number of concave surfaces observed on said scanning electron microscope photograph image
wherein the non-spherical fine particles comprise siloxane units SiO₂ in an amount of 30 - 50 molar %, siloxane units R¹SiO_{1.5} in an amount of 40 - 60 molar % and siloxane units R²R³SiO in an amount of 5 - 20 molar % so as to be a total of 100 molar %, wherein R¹, R² and R³ are each alkyl group with 1 - 4 carbon atoms or phenyl group
and the particles are prepared by a method comprising the steps of:
using silanol group forming silicide SiX₄ in an amount of 30 - 50 molar %, silanol group forming silicide R⁴SiY₃ in an amount of 40 - 60 molar % and silanol group forming silicide R⁵R⁵SiZ₂ in an amount of 5 - 20 molar % so as to be a total of 100 molar %;
generating a silanol compound by causing silanol group forming silicide SiX₄ to contact water in the presence of an acidic catalyst so as to undergo hydrolysis; and
causing a condensation reaction of said silanol compound with silanol group forming silicide R⁴SiY₃ and silanol group forming silicide R⁵R⁶SiZ₂ in an aqueous condition in the presence of an acidic catalyst and a nonionic surfactant;
wherein R⁴, R⁵ and R⁶ are each alkyl group with 1 - 4 carbon atoms or phenyl group, and X, Y and Z are each alkoxy group with 1-4 carbon atoms, alkoxyethoxy group having alkoxy group with 1-4 carbon atoms, acyloxy group with 2-4 carbon atoms, N,N-dialkylamino group having alkyl group with 1-4 carbon atoms, hydroxyl group, halogen atom or hydrogen atom.

2. The non-spherical fine particles of claim 1, wherein the acid catalyst is selected from the group consisting of hydrochloric acid, sulfuric acid, nitric acid, phosphoric acid, formic acid, acetic acid, citric acid, methane sulfonic acid, toluene sulfonic acid, dodecyl benzene sulfonic acid, decyl sulfate, dodecyl sulfate, tetradecyl sulfate and hexadecyl sulfate.

3. The non-spherical fine particles of claim 1, wherein the surfactant is selected from the group consisting of nonionic surfactants with oxyalkylene groups comprising oxyethylene groups and/or oxypropylene groups.

4. The non-spherical fine particles of claim 1 wherein the average number of concave surfaces of which the ratio of the maximum diameter m₁ with respect to the maximum external diameter L₁ is in the range of 0.50 - 0.90 is in the range of 3 - 7 per non-spherical fine particle.

5. The non-spherical fine particles of claims 1 or 2 of which oil absorption is 70 - 170ml/100g.

6. A method of producing the non-spherical fine particles of any of claims 1 to 3, said method comprising the steps of:
using silanol group forming silicide SiX₄ in an amount of 30 - 50 molar %, silanol group forming silicide R⁴SiY₃ in an amount of 40 - 60 molar % and silanol group forming silicide R⁵R⁵SiZ₂ in an amount of 5 - 20 molar % so as to be a total of 100 molar %;
generating a silanol compound by causing silanol group forming silicide SiX₄ to contact water in the presence of an acidic catalyst so as to undergo hydrolysis; and
causing a condensation reaction of said silanol compound with silanol group forming silicide R⁴SiY₃ and silanol group forming silicide R⁵R⁶SiZ₂ in an aqueous condition in the presence of an acidic catalyst and a nonionic surfactant;
wherein R⁴, R⁵ and R⁶ are each alkyl group with 1 - 4 carbon atoms or phenyl group, and X, Y and Z are each alkoxy group with 1-4 carbon atoms, alkoxyethoxy group having alkoxy group with 1-4 carbon atoms, acyloxy group with 2-4 carbon atoms, N,N-dialkylamino group having alkyl group with 1-4 carbon atoms, hydroxyl group, halogen atom or hydrogen atom.

7. A cosmetic material containing the non-spherical fine particles of any of claims 1 to 3 in an amount of 0.1 - 10 mass %.

8. A resin composition containing the non-spherical fine particles of any of claims 1 to 3 in an amount of 0.1 - 10 mass %.

## Patentansprüche

1. Nichtsphärische feine Partikel, die jeweils eine polyedrische allgemeine Form mit sechs oder mehr Oberflächen aufweisen, von denen jede als konkave Oberfläche ausgebildet ist,
wobei die maximalen Außendurchmesser L₁ der nichtsphärischen feinen Partikel einen Mittelwert im Bereich von 0,1 bis 20 µm aufweisen,
wobei die Verhältnisse zwischen den minimalen Außendurchmessern L₂ und den maximalen Außendurchmessern L₁ der einzelnen nichtsphärischen feinen Partikel einen Mittelwert im Bereich von 0,60 bis 0,97 aufweisen,
wobei die nichtsphärischen Partikel pro Partikel eine mittlere Anzahl von 6 bis 14 konkave Oberflächen aufweisen, von denen das Verhältnis des maximalen Durchmessers m₁ in Bezug auf den maximalen Außendurchmesser L₁ im Bereich von 0,20 bis 0,90 liegt, und
wobei die Mittelwerte Werte sind, die von willkürlich ausgewählten 20 einer Rasterelektronenmikroskop-Fotoaufnahme der nichtsphärischen feinen Partikel erhalten werden, und die Anzahl der konkaven Oberflächen pro nichtsphärischem feinen Partikel als doppelt so groß wie die Anzahl der beobachteten konkaven Oberflächen auf der Rasterelektronenmikroskop-Fotoaufnahme definiert ist;
wobei die nichtsphärischen feinen Partikel Siloxan-Einheiten SiO₂ in einer Menge von 30 bis 50 Mol-%, Siloxan-Einheiten R¹SiO_{1.5} in einer Menge von 40 bis 60 Mol-% und Siloxan-Einheiten R²R³SiO in einer Menge von 5 bis 20 Mol-% umfassen, um so insgesamt 100 Mol-% auszumachen, wobei R¹, R² und R³ jeweils eine Alkylgruppe mit 1 bis 4 Kohlenstoffatomen oder eine Phenylgruppe sind;
und wobei die Partikel hergestellt werden mit Hilfe eines Verfahrens, umfassend die Schritte:
Verwenden von Silanol-Gruppe bildendem Silizid SiX₄ in einer Menge von 30 bis 50 Mol-%, Silanol-Gruppe bildendem Silizid R⁴SiY₃ in einer Menge von 40 bis 60 Mol-% und Silanol-Gruppe bildendem Silicid R⁵R⁵-SiZ₂ in einer Menge von 5 bis 20 Mol-%, um so insgesamt 100 Mol-% auszumachen;
Erzeugen einer Silanol-Verbindung, indem bewirkt wird, dass Silanol-Gruppe bildendes Silicid SiX₄ in Gegenwart eines sauren Katalysators mit Wasser in Kontakt kommt, so dass es einer Hydrolyse unterzogen wird; und
Herbeiführen einer Kondensationsreaktion der Silanol-Verbindung mit Silanol-Gruppe bildendem Silizid R⁴SiY₃ und Silanol-Gruppe bildendem Silizid R⁵R⁶-SiZ₂ in einem wässrigen Zustand in Gegenwart eines sauren Katalysators und eines nichtionischen Tensids;
worin R⁴, R⁵ und R⁶ jeweils eine Alkylgruppe mit 1 bis 4 Kohlenstoffatomen oder eine Phenylgruppe sind und X, Y und Z jeweils Alkoxygruppe sind, die 1 bis 4 Kohlenstoffatome aufweist, Alkoxyethoxy-Gruppe sind, die eine Alkoxy-Gruppe mit 1 bis 4 Kohlenstoffatomen aufweist, Acyloxy-Gruppe sind, die 2 bis 4 Kohlenstoffatome aufweist, N,N-Dialkylamino-Gruppe sind, die eine Alkylgruppe mit 1 bis 4 Kohlenstoffatomen aufweist, Hydroxylgruppe, Halogenatom oder Wasserstoffatom sind.

2. Nichtsphärische feine Partikel nach Anspruch 1, wobei der saure Katalysator ausgewählt ist aus der Gruppe bestehend aus Salzsäure, Schwefelsäure, Salpetersäure, Phosphorsäure, Ameisensäure, Essigsäure, Citronensäure, Methansulfonsäure, Toluolsulfonsäure, Dodecylbenzolsulfonsäure, Decylsulfat, Dodecylsulfat, Tetradecylsulfat und Hexadecylsulfat.

3. Nichtsphärische feine Partikel nach Anspruch 1, wobei das Tensid ausgewählt ist aus der Gruppe bestehend aus nichtionischen Tensiden mit Oxyalkylen-Gruppen, die Oxyethylen-Gruppen und/oder Oxypropylen-Gruppen.

4. Nichtsphärische feine Partikel nach Anspruch 1, wobei die mittlere Anzahl von konkaven Oberflächen, deren Verhältnis des maximalen Durchmessers m₁ in Bezug zu dem maximalen Außendurchmesser L₁ im Bereich von 0,50 bis 0,90 liegt, pro nichtsphärisches feines Partikel im Bereich von 3 bis 7 liegt.

5. Nichtsphärische feine Partikel nach Anspruch 1 oder 2, deren Ölabsorption 70 bis 170 ml/100 g beträgt.

6. Verfahren zum Herstellen der nichtsphärischen feinen Partikel nach einem der Ansprüche 1 bis 3, wobei das Verfahren die Schritte umfasst:
Verwenden von Silanol-Gruppe bildendem Silizid SiX₄ in einer Menge von 30 bis 50 Mol-%, Silanol-Gruppe bildendem Silizid R⁴SiY₃ in einer Menge von 40 bis 60 Mol-% und Silanol-Gruppe bildendem Silicid R⁵R⁵-SiZ₂ in einer Menge von 5 bis 20 Mol-%, um so insgesamt 100 Mol-% auszumachen;
Erzeugen einer Silanol-Verbindung, indem bewirkt wird, dass Silanol-Gruppe bildendes Silicid SiX₄ in Gegenwart eines sauren Katalysators mit Wasser in Kontakt kommt, so dass es einer Hydrolyse unterzogen wird; und
Herbeiführen einer Kondensationsreaktion der Silanol-Verbindung mit Silanol-Gruppe bildendem Silizid R⁴SiY₃ und Silanol-Gruppe bildendem Silizid R⁵R⁶-SiZ₂ in einem wässrigen Zustand in Gegenwart eines sauren Katalysators und eines nichtionischen Tensids;
worin R⁴, R⁵ und R⁶ jeweils eine Alkylgruppe mit 1 bis 4 Kohlenstoffatomen oder eine Phenylgruppe sind und X, Y und Z jeweils Alkoxygruppe sind, die 1 bis 4 Kohlenstoffatome aufweist, Alkoxyethoxy-Gruppe sind, die eine Alkoxy-Gruppe mit 1 bis 4 Kohlenstoffatomen aufweist, Acyloxy-Gruppe sind, die 2 bis 4 Kohlenstoffatome aufweist, N,N-Dialkylamino-Gruppe sind, die eine Alkylgruppe mit 1 bis 4 Kohlenstoffatomen aufweist, Hydroxylgruppe, Halogenatom oder Wasserstoffatom sind.

7. Kosmetisches Material, enthaltend die nichtsphärischen feinen Partikel nach einem der Ansprüche 1 bis 3 in einer Menge von 0,1 bis 10 Masse-%.

8. Harzzusammensetzung, enthaltend die nichtsphärischen feinen Partikel nach einem der Ansprüche 1 bis 3 in einer Menge von 0,1 bis 10 Masse-%.

## Revendications

1. Particules fines non sphériques ayant chacune une forme générale polyhédrique avec six surfaces ou plus, dont chacune est formée en tant que surface concave,
dans lesquelles les diamètres externes maximaux L₁ des particules fines non sphériques ont une valeur moyenne dans la plage de 0,1 à 20 µm,
dans lesquelles les ratios entre les diamètres externes minimaux L₂ et les diamètres externes maximaux L₁ des particules fines non sphériques individuelles ont une valeur moyenne dans la plage de 0,60 à 0,97,
dans lesquelles lesdites particules fines non sphériques ont un nombre moyen par particule de 6 - 14 surfaces concaves dont le ratio du diamètre maximum m₁ par rapport au diamètre externe maximal L₁ se situe dans la plage de 0,20 à 0,90, et
dans lesquelles les valeurs moyennes sont des valeurs obtenues à partir de 20 sélectionnées arbitrairement d'une image photographique de microscope électronique à balayage desdites particules fines non sphériques et le nombre de surfaces concaves par particule fine non sphérique est défini comme étant le double du nombre de surfaces concaves observées sur ladite image photographique de microscope électronique à balayage,
dans lesquelles les particules fines non sphériques comprennent des motifs siloxane SiO₂ en une quantité de 30 à 50 % en moles, des motifs siloxane R¹SiO_{1.5} en une quantité de 40 à 60 % en moles et des motifs siloxane R²R³SiO en une quantité de 5 - 20 % en moles de façon à représenter un total de 100 % en moles, dans lesquelles R¹, R² et R³ sont chacun un groupe alkyle avec 1 à 4 atomes de carbones ou un groupe phényle,
et les particules sont préparées par un procédé comprenant les étapes consistant à :
utiliser un siliciure SiX₄ formant un groupe silanol en une quantité de 30 à 50 % en moles, un siliciure R⁴SiY₃ formant un groupe silanol en une quantité de 40 à 60 % en moles et un siliciure R⁵R⁵SiZ₂ formant un groupe silanol en une quantité de 5 à 20 % en moles de façon à représenter un total de 100 % en moles ;
générer un composé au silanol en amenant le siliciure SiX₄ formant un groupe silanol à entrer en contact avec de l'eau en présence d'un catalyseur acide de manière à subir une hydrolyse ; et
provoquer une réaction de condensation dudit composé au silanol avec un siliciure R⁴SiY₃ formant un groupe silanol et un siliciure R⁵R⁶SiZ₂ formant un groupe silanol dans une condition aqueuse en présence d'un catalyseur acide et d'un tensioactif non ionique ;
dans lesquelles R⁴, R⁵ et R⁶ sont chacun un groupe alkyle avec 1 à 4 atomes de carbone ou un groupe phényle, et X, Y et Z sont chacun un groupe alcoxy avec 1 à 4 atomes de carbone, un groupe alcoxyéthoxy ayant un groupe alcoxy avec 1 à 4 atomes de carbone, un groupe acyloxy ayant 2 à 4 atomes de carbone, un groupe N,N-dialkylamino ayant un groupe alkyle avec 1 à 4 atomes de carbone, un groupe hydroxyle, un atome d'halogène ou un atome d'hydrogène.

2. Particules fines non sphériques selon la revendication 1, dans lesquelles le catalyseur acide est sélectionné dans le groupe constitué par l'acide chlorhydrique, l'acide sulfurique, l'acide nitrique, l'acide phosphorique, l'acide formique, l'acide acétique, l'acide citrique, l'acide méthanesulfonique, l'acide toluènesulfonique, l'acide dodécylbenzènesulfonique, le sulfate décylique, le sulfate dodécylique, le sulfate tétradécylique et le sulfate hexadécylique.

3. Particules fines non sphériques selon la revendication 1, dans lesquelles le tensioactif est sélectionné dans le groupe constitué de tensioactifs non ioniques avec des groupes oxyalkylène comprenant des groupes oxyéthylène et/ou des groupes oxypropylène.

4. Particules fines non sphériques selon la revendication 1, dans lesquelles le nombre moyen de surfaces concaves dont le ratio du diamètre maximal m₁ par rapport au diamètre externe maximal L₁ se situe dans la plage de 0,50 à 0,90 se situe dans la plage de 3 à 7 par particule fine non sphérique.

5. Particules fines non sphériques selon les revendications 1 ou 2 dont l'absorption d'huile est de 70 à 170 ml/100 g.

6. Procédé de production de particules fines non sphériques selon l'une quelconque des revendications 1 à 3, ledit procédé comprenant les étapes consistant à :
utiliser un siliciure SiX₄ formant un groupe silanol en une quantité de 30 à 50 % en moles, un siliciure R⁴SiY₃ formant un groupe silanol en une quantité de 40 à 60 % en moles et un siliciure R⁵R⁵SiZ₂ formant un groupe silanol en une quantité de 5 à 20 % en moles de façon à représenter un total de 100 % en moles ;
générer un composé au silanol en amenant le siliciure SiX₄ formant un groupe silanol à entrer en contact avec de l'eau en présence d'un catalyseur acide de manière à subir une hydrolyse ; et
provoquer une réaction de condensation dudit composé au silanol avec un siliciure R⁴SiY₃ formant un groupe silanol et un siliciure R⁵R⁶SiZ₂ formant un groupe silanol dans une condition aqueuse en présence d'un catalyseur acide et d'un tensioactif non ionique ;
dans lesquelles R⁴, R⁵ et R⁶ sont chacun un groupe alkyle avec 1 à 4 atomes de carbone ou un groupe phényle, et X, Y et Z sont chacun un groupe alcoxy avec 1 à 4 atomes de carbone, un groupe alcoxyéthoxy ayant un groupe alcoxy avec 1 à 4 atomes de carbone, un groupe acyloxy ayant 2 à 4 atomes de carbone, un groupe N,N-dialkylamino ayant un groupe alkyle avec 1 à 4 atomes de carbone, un groupe hydroxyle, un atome d'halogène ou un atome d'hydrogène.

7. Substance cosmétique contenant les particules fines non sphériques selon l'une quelconque des revendications 1 à 3 en une quantité de 0,1 à 10 % en masse.

8. Composition de résine contenant les particules fines non sphériques selon l'une quelconque des revendications 1 à 3 en une quantité de 0,1 à 10 % en masse.
